(19) Europäisches Patentamt
European Patent Office
Office européen des brevets

(11) Veröffentlichungsnummer: **0 243 901**
**A1**

(12) **EUROPÄISCHE PATENTANMELDUNG**

(21) Anmeldenummer: 87106028.1

(22) Anmeldetag: 24.04.87

(51) Int. Cl.³: C 12 Q 1/26
C 12 Q 1/28, C 12 Q 1/34

(30) Priorität: 28.04.86 BE 216599
29.08.86 BE 217095

(43) Veröffentlichungstag der Anmeldung:
04.11.87 Patentblatt 87/45

(84) Benannte Vertragsstaaten:
ES GR

(71) Anmelder: Boehringer Mannheim GmbH
Sandhofer Strasse 116
D-6800 Mannheim 31(DE)

(72) Erfinder: Delanghe, Joris R.
Assesteenweg 128
B-1742 Ternat(BE)

(72) Erfinder: De Buyzere, Marc L.
Sint-Pietersgroenestraat 129
B-8310 Brugge 1(BE)

(72) Erfinder: De Scheerder, Ivan K.
Krijgslaan 131
B-9000 Gent(BE)

(72) Erfinder: Siedel, Joachim
Bahnhofstrasse 6
D-8131 Bernried(DE)

(72) Erfinder: Staepels, Johnny
Jahnstrasse 20
D-8130 Starnberg(DE)

(74) Vertreter: Fouquet, Herbert et al,
Boehringer Mannheim GmbH Sandhofer Strasse 116
Postfach 31 01 20
D-6800 Mannheim 31(DE)

(54) **Verfahren und Reagenz zum Nachweis von Schädigungen bzw. Erkrankungen des Herzens.**

(57) Zum spezifischen und frühen Nachweis von Schädigungen bzw. Erkrankungen des Herzens, insbesondere von akutem Herzinfarkt, wird ein niedermolekularer Metabolit, beispielsweise Creatin oder Creatinphosphat, 2 - 8 Stunden nach dem Auftreten der ersten Symptome bestimmt. Zur Bestimmung des hierbei enzymatisch freigesetzten $H_2O_2$ wird ein Reagenz, das neben einem Elektronen- oder Radikal-Akzeptor ein Anilinderivat als Wasserstoff-Donor beinhaltet, verwendet.

EP 0 243 901 A1

Croydon Printing Company Ltd.

# Verfahren und Reagenz zum Nachweis von Schädigungen bzw. Erkrankungen des Herzens

Die Erfindung betrifft ein Verfahren und ein Reagenz zum Nachweis von Schädigungen bzw. Erkrankungen des Herzmuskels, wie z. B. Herzinfarkt, sowie dessen Verwendung zur Verlaufskontrolle bei Herzoperationen, zur weiteren Beobachtung nach einem Herzinfarkt und bei bestimmten Indikationen zur Herztherapie, wie z. B. bei thrombolytischen Behandlungen.

Zur Erkennung von Schädigungen bzw. Erkrankungen des Herzens, insbesondere von Herzinfarkt, werden heute in der klinischen Praxis neben der Beurteilung der persönlichen Krankengeschichte zwei grundlegende Prinzipien herangezogen. Diese beruhen entweder auf Störungen elektrischer Potentiale des Herzmuskels oder auf Veränderungen der Enzymaktivitäten im Blut. Aus elektrokardiographischen Befunden (EKG) können Informationen über die unterschiedlichen Entwicklungsstadien und die Lokalisation eines Infarktes gewonnen werden. Die Veränderungen der Enzymaktivitäten, die auf Herzschädigungen, beispielsweise einen Herzinfarkt hinweisen, stützen sich insbesondere auf die Messung der Erhöhung des Gehalts muskelspezifischer Enzyme im Serum, die aus zugrundegegangenen Herzmuskelzellen resultieren. Dabei spielen die Bestimmung der Creatinkinase (CK) bzw. die des CK-Isoenzyms (CK-MB) und die der Lactatdehydrogenase (LDH) und Glutamat-Oxalacetat-Transaminase (GOT) in der klinischen Praxis eine dominierende Rolle.

Das Enzym Creatinkinase (ATP: Creatin-Phosphotransferase, E.C. 2.7.3.2) bzw. dessen Isoenzyme kommen bei den Vertebraten in besonders hohen Konzentrationen im Muskel- und Nervengewebe vor. Dort katalysiert CK die Umwandlung von Creatin in Creatinphosphat, welches die Speicherform für energiereiche Phosphate in diesem Gewebe darstellt. Creatinphosphat geht nach Dephosphorylierung über Creatin in einer nicht-enzymatischen, irreversiblen Reaktion in Creatinin über. Das Creatinin stellt das katabole Endprodukt des Creatin-Stoffwechsels der Säuger dar und wird aus dem Blut über die Niere im Urin ausgeschieden.

Die Bestimmung der Creatinkinase im Serum als Methode zur Erkennung von Erkrankungen der Skelett- und Herzmuskulatur, speziell von Herzinfarkt, resultiert aus einer vermehrten Enzymfreisetzung nach Schädigung der Skelett- und Herzmuskulatur. Schon nach 6 - 8 Stunden nach dem Herzinfarkt kann ein Anstieg der CK-Aktivität beobachtet werden, das Maximum (10 - 20faches der Norm) wird nach etwa 18 - 24 Stunden erreicht.

Der Anstieg der Aktivität der Lactatdehydrogenase bzw. der herzmuskelspezifischen LDH-Isoenzyme im Serum basiert gleichfalls auf geschädigtem bzw. zugrundegegangenem Herzmuskelgewebe. Sichere Erhöhungen werden in 90 % der Fälle 12 Stunden nach Beginn der klinischen Symptomatologie gefunden. Das Maximum des LDH-Anstiegs (2 - 10faches der Norm) liegt zwischen 48 und 72 Stunden.

Häufig wird in der klinischen Diagnostik von Herzerkrankungen außerdem die Bestimmung der GOT-Aktivität als zusätzlicher Parameter herangezogen. Der Anstieg der GOT-Aktivität erfolgt zeitlich ungefähr parallel zu dem der CK-Aktivität.

Ein Nachteil der Bestimmung der genannten Enzyme zum Nachweis von Herzinfarkt und anderen Herzmuskelschädigungen ist jedoch, daß eine signifikante Erhöhung der Enzymaktivität im Blut bzw. Serum erst ungefähr 8 Stunden nach Eintritt der Schädigung auftritt, wobei das Maximum nach etwa 24 Stunden erreicht wird. Auch entsprechende Elektrokardiogramme liefern in ca. 15 % der Fälle keine eindeutigen, verläßlichen Aussagen. Die bekannten Verfahren sind

demnach für die Verwendung zur frühen spezifischen Diagnose des Herzinfarkts oder anderen Schädigungen des Herzmuskelgewebes nur beschränkt geeignet. Nur eine möglichst früh, d. h. bis zu etwa 4 Stunden nach dem Ereignis einsetzende, gezielte Therapie aufgrund einer spezifischen Diagnose kann jedoch, insbesondere beim akuten Herzinfarkt, einen therapeutischen Effekt erzielen.

Der Erfindung lag daher die Aufgabe zugrunde, ein einfaches, schnell durchzuführendes spezifisches Verfahren zum Nachweis von Herzinfarkt und anderen Schädigungen des Herzmuskels zu entwickeln, wodurch die Aussagekraft der Herzinfarkt-Diagnostik erhöht wird.

Diese Aufgabe wird erfindungsgemäß dadurch gelöst, daß mindestens ein niedermolekularer Metabolit, beispielsweise Creatin (Molekulargewicht 131 $g \cdot mol^{-1}$) oder Creatinphosphat, in der Probe einer Körperflüssigkeit, wie Urin, Blut, Serum, Plasma, Speichel und ihre Ultrafiltrate oder von Gewebeextrakten bestimmt wird.

Bei den Schädigungen oder Erkrankungen des Herzens handelt es sich beispielsweise am häufigsten um die Formen akuter Herzinfarkt, Mikronekrosen, akute und stumme Myokardischämie, Herzkontusion nach stumpfen Thoraxtraumen.

Mit dem Begriff Metabolite sind in dem hier beschriebenen Zusammenhang die aus dem Creatin-Stoffwechsel resultierenden oder die in diesem Stoffwechsel umgesetzten Produkte sowie mögliche biologische und chemische Vor- und Folgeprodukte und künstliche Artefakte zu verstehen. Beispiele stellen die folgenden Substrate dar: Creatin, Creatinphosphat, Creatinin, Sarcosin, Phosphoenolpyruvat, Pyruvat, Lactat, Harnstoff, Glycin, Formaldehyd, Aminosäuren des Herzmuskels sowie deren biochemischen und chemischen Umwandlungsprodukte, die aufgrund ihres geringen Molekulargewichts bei Schädigungen des Herzmuskelgewebes vermehrt freigesetzt werden können.

Creatin, das aus Glycin und Arginin überwiegend in der Leber und in der Niere synthetisiert und vorwiegend als Creatinin ausgeschieden wird, liegt beim gesunden Menschen bzw. Säugetier nur in geringen Konzentrationen im Serum bzw. Harn vor (ca. 5 - 10 mg/l bzw. 2 - 4 mg/l).

Die Bestimmung des Creatins wurde bisher, im Gegensatz zur Creatinin-Bestimmung, im wesentlichen für die klinisch-chemische Forschung herangezogen. Beispielsweise wird ein vermehrter Creatingehalt des Blutes (Creatinämie) bei zunehmender Niereninsuffizienz beobachtet (siehe hierzu z. B. JP 58.009.699). Des weiteren wird bei Patienten mit hämolytischen Anämien während der schmerzhaften hämolytischen Krise ein um das 10 bis 20fache gesteigerter Creatinspiegel im 24 Stunden-Urin festgestellt (Beyer et al., Clin. Chem. 31, 1232 - 1234 (1985)). Außerdem wird bei verschiedenen Erkrankungen der Skelettmuskulatur ein erhöhter Creatinspiegel im Serum und im Urin beobachtet (Yasuhara et al., Clin. Chim. Acta 122, 181 - 188 (1982); Suzuki und Yoshida, Clin. Chim. Acta 140, 289 - 294 (1984)). Neu ist hingegen, daß das sehr schnelle Auftreten eines erhöhten Creatinspiegels in Körperflüssigkeiten, wie insbesondere im Urin zur Diagnose von Herzerkrankungen und Herzschädigungen herangezogen werden kann.

Der erfindungsgemäße Effekt, d. h. die Erkenntnis des Zusammenhangs des erhöhten Metabolitspiegels in Körperflüssigkeiten mit Herzschädigungen und Herzerkrankungen ist sehr überraschend. Ein solcher Zusammenhang ist bisher nicht bekannt geworden. Hierdurch werden möglicherweise zusätzlich Energiereserven unter Umwandlung von Creatinphosphat in Creatin und gleichzeitiger ATP-Bildung mobilisiert. Im Gegensatz zum Anstieg der CK-Aktivität, der im Blut/Serum erst nach 8 - 24 Stunden auftritt, zeigt sich nach der Erfindung aber, daß der Anstieg eines niedermolekularen Metaboliten, wie z. B. des Creatins, im Blut bzw. Urin bereits nach ungefähr einer Stunde eintritt, mit einem Maximum nach etwa 3 Stunden. Somit stellt die Bestimmung eines niedermolekularen Metaboliten zum Nachweis eines akuten Herzinfarkts oder anderer Schädigungen des Herzmuskels oder Herzerkrankungen ein völlig neues Prinzip in der biochemischen Herzdiagnostik dar.

Der jeweilige Metabolit kann in der Probe einer Körperflüssigkeit entweder enzymatisch über gekoppelte Enzymreaktionen oder über rein chemische Reaktionen bestimmt werden, wobei der photometrische Nachweis bevorzugt ist. Creatin kann beispielsweise in einer gekoppelten Reaktion mit Creatinkinase, Pyruvatkinase und Lactatdehydrogenase und der Messung des letztlich entstehenden $NAD^+$ im UV-Bereich (in: H. U. Bergmeyer, Methods of Enzymatic Analysis, 3th edn. New York, Academic Press, 1985, Vol. 8, 500 - 514) bzw. nach weiterer, dem Fachmann bekannter Umsetzung als entsprechender Farbstoff bestimmt werden. Die Bestimmung von Creatin unter Verwendung der Enzyme Creatinase (Creatinamidinohydrolase, E.C.3.5.3.3.) und Sarcosinoxidase (bzw. Sarcosindehydrogenase) und Messung von gebildetem $H_2O_2$ oder verbrauchtem $O_2$ (bzw. gebildetem NADH oder verbrauchtem $NAD^+$) bzw. Formaldehyd möglich. Bevorzugt wird beim erfindungsgemäßen Verfahren die Bestimmung mittels Sarcosinoxidase und Messung von gebildetem $H_2O_2$ unter Katalyse von Peroxidase (oder peroxidatisch wirksamen Substanzen) und einem farbbildenden System. Es besteht jedoch außerdem die Möglichkeit, gebildetes $H_2O_2$ oder verbrauchtes $O_2$ titrimetrisch, potentiometrisch sowie polarographisch nachzuweisen. Auch diese Verfahren sind dem Fachmann bekannt.

Die erfindungsgemäße Bestimmung des Metaboliten, z. B. des Creatins, bei Herzschädigungen wie Herzinfarkt, erfolgt im Zeitraum von etwa 1 bis 24 Stunden, vorzugsweise im Zeitraum von 2 bis 8 Stunden, mit einem Maximum bei etwa 3 Stunden nach Eintritt der Herzschädigung. Die Messung bis zu 24 Stunden nach dem Auftreten des akuten Herzinfarkts kann zur weiteren medizinischen Beobachtung, wie z. B. zur Absicherung der CK-abhängigen Diagnostik oder zur Erkennung eventuell auftretender Re-Infarkte dienen (siehe Fig. 1 und 2).

Als Körperflüssigkeit für die Bestimmung des Creatins wird bevorzugt Urin herangezogen, da hier der Anstieg des Creatins signifikant höher liegt. Für die Bestimmung des Creatins wird vorteilhaft zum Nachweis von Herzschädigungen die enzymatische Methode, die unter Zusatz von Creatinamidinohydrolase (Creatinase) und

Sarcosinoxidase (E.C.1.5.3.1) aus Creatin letztlich gebildetes Wasserstoffperoxid mit Peroxidase und einem Farbbildungsreagenz zu einem Farbstoff umsetzt, verwendet. Anstatt Sarcosinoxidase kann auch Sarcosindehydrogenase eingesetzt werden und NADH und/oder Formaldehyd bestimmt werden.

Die folgende Erkenntnis stellt hierbei einen weiteren wichtigen und nicht a priori als notwendig vorhersehbaren Teil der Erfindung dar. Diese beruht auf dem überraschenden Befund, daß Sarcosin unter den spezifischen Bedingungen einer Herzmuskelschädigung in größerer, gegenüber Creatin ins Gewicht fallender, Menge im Blut bzw. Urin auftritt, denn bei gesunden Menschen kommt Sarcosin - wenn überhaupt - nur in Spuren und gegenüber Creatin in vernachlässigbaren Konzentrationen im Blut bzw. Urin vor. Ein erhöhter Sarcosinspiegel im Zusammenhang mit Herzerkrankungen, wie z. B. Herzinfarkt, ist ebenfalls bislang nicht bekannt. Es ist somit vorteilhaft, für jeden Bestimmungsansatz eine weitere Umsetzung entweder vorgeschaltet oder parallel durchzuführen. Diese zusätzliche Bestimmung läuft identisch ab, allerdings ohne die Anwesenheit von Creatinamidinohydrolase (Creatinase). Im Anschluß wird aus der Differenz der Meßsignale die wahre Creatinkonzentration berechnet. Dadurch stimmen die erhaltenen Creatinwerte mit der Referenzmethode (High Performance Liquid Chromatographie = HPLC) überein und erlauben eine zuverlässige Aussage. Um also sicherzustellen, daß die Intensität des gebildeten Farbstoffs einzig auf die Menge an Creatin bzw. hieraus gebildetes Wasserstoffperoxid und nicht auf eventuell vorhandenes Sarcosin oder andere mit Sarcosinoxidase reagierende Substanzen in der Probe zurückzuführen ist, wird für jede Creatinbestimmung neben dem allgemein üblichen Leerwert (Wasser oder Pufferlösung anstatt Probe) ein sogenannter Sarcosin-Leerwert berücksichtigt, der sich durch analoges Vorgehen bei der Bestimmung ohne den Zusatz einer Creatinase ergibt. Der Nettogehalt an Creatin wird durch den Abzug dieses Leerwertes von der ersten Messung erhalten.

Bei der Bestimmung des Creatinphosphats wird analog verfahren. Zu jeder Umsetzung, bei der in Gegenwart von Creatinkinase (und ADP), Creatinamidinohydrolase und Sarcosinoxidase aus Creatinphosphat $H_2O_2$ entsteht (das in einer entsprechenden Folgereaktion photometrisch quantifiziert wird), wird ein weiterer Bestimmungsansatz ohne Creatinkinase (und ADP) vorgeschaltet oder parallel durchgeführt. Hierbei wird aus Creatin und Sarcosin gebildetes $H_2O_2$ erfaßt. Der Nettogehalt des Creatinphosphats in der Probe wird durch Differenzbildung der beiden Bestimmungswerte ermittelt.

Ein weiterer Gegenstand der Erfindung ist ein für das erfindungsgemäße Verfahren, vor allem für die Creatin-Bestimmung, geeignetes Farbbildungsreagenz zum Nachweis des in der Sarcosinoxidase-Reaktion gebildeten Wasserstoffperoxids. In der Literatur sind zahlreiche Verbindungen beschrieben, die als Indikatoren für den Nachweis von Wasserstoffperoxid mit Peroxidase als Katalysator eingesetzt werden können. Solche Indikatoren sind: Benzidin und Benzidin-Derivate, verschiedene Phenole, Polyphenole, wie z. B. Guajakharz, Leukofarbstoffe, wie z. B. Leukomalachitgrün, Dichlorphenolindophenol, Aminocarbazole, Triarylimidazole, 2,2'-Azino-di-[3-äthyl-benzthiazolsulfonsäure-(6)] sowie Farbstoffsysteme, welche einen Wasserstoff-Donor und einen Elektronen- oder Radikal-Akzeptor enthalten. Letzere haben sich hierbei besonders bewährt. Bei dem erfindungsgemäßen Farbbildungsreagenz haben sich als Wasserstoff-Donoren insbesondere Anilin-Derivate als geeignet erwiesen. Bevorzugt sind dabei Anilin-Derivate der folgenden Formel:

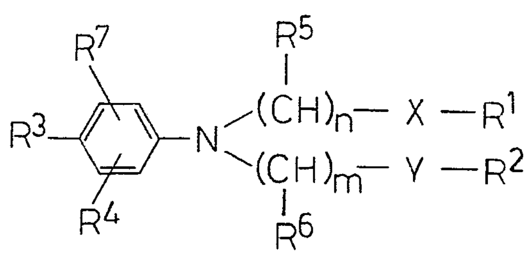

in der

| | |
|---|---|
| n und m | die Zahlen von 1 bis 3 bedeuten können, |
| X und Y | einen Valenzstrich, einen Arylen- oder Hydroxyalkylenrest mit 1 - 6 C-Atomen, |
| $R^1$ und $R^2$ | Wasserstoff, eine Hydroxy-, Alkyl- oder Alkoxygruppe mit 1 - 6 C-Atomen, eine Carboxyl-, Sulfonsäure-, eine Acetamido- oder eine P(O)(OR)(R')-Gruppe sein kann, |

worin

| | |
|---|---|
| P | ein Phosphoratom bedeutet, |
| R | Wasserstoff oder eine Alkylgruppe mit 1 - 6 C-Atomen und |
| R' | eine Hydroxy-, eine Aryl-, Alkyl- oder Alkoxygruppe mit 1 - 6 C-Atomen sein kann, |
| $R^3$ | Wasserstoff, Halogen, eine Carboxyl- oder eine Sulfonsäuregruppe sein kann, |

wobei Halogen, Jod, Brom und bevorzugt Chlor und Fluor umfaßt,

| | |
|---|---|
| $R^4$ und $R^7$ | Wasserstoff, eine Alkoxy- oder Alkylgruppe mit 1 - 6 C-Atomen, |
| $R^5$ und $R^6$ | für Wasserstoff, eine Aryl- oder Alkylgruppe mit 1 - 6 C-Atomen stehen und |
| $R^5$ und $R^7$ | bzw. $R^6$ und $R^4$ für den Fall, daß die beiden Substituenten orthoständig zueinander stehen, zusammen eine gesättigte oder ungesättigte Kohlenwasserstoffkette mit 2 - 6 C-Atomen bilden können, die durch Hydroxy- oder Oxogruppen substituiert sein kann, |

sowie ihre Salze mit Säuren und Basen.

Bevorzugte Anilin-Derivate sind insbesondere (N-Methylanilino)-methanphosphonsäure, N-Ethyl-N-(2-hydroxy-3-sulfopropyl)-m-toluidin (TOOS), N-Ethyl-N-sulfopropylanilin (ALPS), N-Ethyl-N-sulfopropyl-m-toluidin (TOPS), N-Ethyl-N-sulfopropyl-m-anisidin (ADPS), N-Ethyl-N-sulfopropyl-3,5-dimethylanilin (MAPS), N-Ethyl-N-sulfopropyl-3,5-dimethoxyanilin (DAPS), N-Sulfopropyl-3,5-dimethoxyanilin (HDAPS), N-Ethyl-N-(2-hydroxy-3-sulfopropyl)anilin (ALOS), N-Ethyl-N-(2-hydroxy-3-sulfopropyl)-m-anisidin (ADOS), N-Ethyl-N-(2-hydroxy-3-sulfopropyl)-3,5-dimethylanilin (MAOS), N-Ethyl-N-(2-hydroxy-3-sulfopropyl)-3,5-dimethoxyanilin (DAOS), N-(2-Hydroxy-3-sulfopropyl)-3,5-dimethoxyanilin (HDAOS) und N-Sulfopropylanilin (HALPS), N-Ethyl-N-hydroxyethyl-m-toluidin (EHT), N-Ethyl-N-(2-sulfoethyl)-m-toluidin (EST), N-Ethyl-N-(3-sulfobenzyl)-m-toluidin (ETTS), N-(2-Acetamidoethyl)-N-ethyl-4-fluor-3-methyl-anilin (F-EMAE) und entsprechende Chinolinderivate, wie z. B. N-Hydroxy-ethyl-1,2,3,4-tetrahydrochinolin (HETC) oder 1,2,3,4-Tetrahydrochinoline-N-methanphosphonsäure (THCMP). Die Verwendung dieser oder verwandter Anilinderivate als Kupplungskomponente in oxidativen Farbbildungsreaktionen zum Nachweis von $H_2O_2$ ist beispielsweise in den folgenden Offenlegungsschriften beschrieben (DE-OS 31 24 594, DE-OS 34 13 693, DE-OS 34 25 219, EP-OS 0 175 250).

Außerdem können z. B. die Phenolderivate 2,4,6-Tribrom-3-hydroxybenzoesäure (TBHB) und 2,4,6-Trijod-3-hydroxybenzoesäure für das erfindungsgemäße Verfahren verwendet werden.

Als Elektronen- und Radikal-Akzeptoren kommen beispielsweise 4-Aminophenazon (4-AAP), 3-Methyl-2-benzothiazolinonhydrazon (MBTH) und 3-Methyl-2-benzothiazolinonhydrazon-6-sulfonsäure (S-MBTH) in Frage, jedoch können auch andere für ihre oxidative Kupplungsfähigkeit bekannte Substanzen in gleicher Weise eingesetzt werden.

Als besonders bevorzugt hat sich die Kupplung von 4-Aminophenazon (4-AAP) oder 3-Methyl-2-benzothiazolinonhydrazon-6-sulfonsäure (S-MBTH) mit N-Ethyl-N-(2-hydroxy-3-sulfopropyl)-m-toluidin (TOOS) oder (N-Methylanilino)-methanphosphonsäure bzw. S-MBTH mit TBHB erwiesen, da hier die Störanfälligkeit gegenüber Urin- bzw. Serumbestandteilen bei der oxidativen Kupplung besonders gering sind. Ein wesentlicher Teil der vorliegenden Erfindung besteht darin, erkannt zu haben, daß die anilinischen Kupplungskomponenten, wie TOOS den phenolischen Kupplungskomponenten, wie TBHB in überraschend hohem Maß, insbesondere in Kombination mit 4-AAP, überlegen sind.

Das erfindungsgemäße Reagenz kann in gelöster oder trockener Form vorliegen. Es kann auf einem geeigneten Träger, z. B. einer Folie, mit Sarcosin-Vorreaktionszone imprägniert enthalten sein. Ein diagnostisches Mittel in Form eines Teststreifens läßt sich herstellen, indem man vorzugsweise Filterpapier, Cellulose oder Kunstfaservlies mit Lösungen der erforderlichen, üblicherweise zur Herstellung von Teststreifen verwendeten Reagenzien in leicht flüchtigen Lösungsmitteln, wie Aceton imprägniert. Dies kann in einem oder mehreren Imprägnierschritten erfolgen. Die fertigen Testpapiere können als solche verwendet werden oder in an sich bekannter Weise an Griffen angeklebt oder vorzugsweise zwischen Kunststoffen und feinmaschigem Netzwerk eingesiegelt werden.

Die Erfindung wird anhand der folgenden Untersuchungsergebnisse und Beispiele näher erläutert:

## I. Medizinischer Teil

### a) Referenzwerte

Zunächst wurde der Creatingehalt im Serum und im Urin von gesund erscheinenden Personen auf die im Beschreibungsteil erwähnte Weise enzymatisch bestimmt. Für die Serumcreatinwerte wurden 138 Personen, aufgeteilt nach Alter und Geschlecht, herangezogen. Die Blutentnahme erfolgte bei allen Personen nach einer fünftägigen Eiweißdiät. Für männliche und weibliche Personen im Alter zwischen 46 und 85 Jahren wurde hierbei ein Konzentrationsbereich von 5,7 - 10,4 mg/l Creatin im Serum erhalten (siehe Tab. 1).

Die Bestimmung des Creatingehalts im Urin ergab einen Mittelwert von 2,7 mg/24 h mit einer Standardabweichung des Mittelwerts von 1,2 mg/24 h.

### b) Ergebnisse bei Herzinfarktpatienten

Bei 22 Patienten mit eindeutigen Herzinfarktsymptomen wurde der Krankheitsverlauf elektrokardiographisch und durch übliche Blutanalytik verfolgt. Im Serum wurde bei diesen Patienten eine maximale Konzentration von 12,5 mg/l mit einer Standardabweichung des Mittelwerts von 3,2 mg/l ca. 3 Stunden nach Einsetzen des retrosternalen Schmerzes gemessen. Im Urin wurden dagegen scharfe Peaks mit Konzentrationen zwischen 100 und 500 mg/l gemessen. Der Urinpeak tritt nur geringfügig später als der Serumpeak auf. Rechnet man diese Daten auf eine 24-Stunden-Ausscheidung im Urin um, so ergibt sich ein Durchschnittswert von 94 mg/l mit einer Standardabweichung des Mittelwerts von 43. Die Figuren 1 und 2 zeigen ein typisches Verhalten der CK- bzw. CK-MB-Enzymaktivitäten und von Creatin im Urin und im Serum.

Außerdem wurde zum Vergleich der Creatin- bzw. der Creatinphosphatgehalt und die CK-Aktivität im Skelettmuskel (n = 3) sowie im gesunden (n = 3) und im Herzinfarkt-geschädigten Herzmuskelgewebe (n =3) bestimmt und jeweils der relative, prozentuale Substanzverlust im geschädigten Herzmuskelgewebe berechnet, der für die drei untersuchten Verbindungen im Mittel ungefähr 60 % beträgt (siehe Tab. 2).

| Altersklasse (Jahre) | n | männlich (mg/l) | n | weiblich (mg/l) |
|---|---|---|---|---|
| 15 - 25 | 4 | 4.27 ± 1.90 | 6 | 6.71 ± 2.84 |
| 26 - 35 | 12 | 4.85 ± 1.67 | 13 | 6.17 ± 2.15 |
| 36 - 45 | 16 | 5.89 ± 1.98 | 11 | 6.98 ± 1.80 |
| 46 - 55 | 13 | 5.80 ± 0.10 | 12 | 6.48 ± 3.06 |
| 56 - 65 | 14 | 5.62 ± 1.57 | 12 | 6.94 ± 2.97 |
| 66 - 75 | 10 | 6.44 ± 2.17 | 6 | 6.14 ± 1.56 |
| 76 - 85 | 5 | 6.09 ± 0.55 | 4 | 9.16 ± 1.32 |

Tab. 1: Creatingehalt im Serum (Referenzwerte)
In den Serumproben von 138 gesunden Personen ($n_{Ges.}$ = 138), differenziert nach Alter und Geschlecht, wurde der Creatingehalt enzymatisch bestimmt.

| | n | CK (U/g*) | Creatin (mg/g*) | Creatinphosphat (mg/g*) |
|---|---|---|---|---|
| Skelettmuskel | 3 | 2809 ± 350 | 1.8 ± 0.5 | 2.3 ± 0.6 |
| Normaler Herzmuskel | 3 | 1186 ± 360 | 1.1 ± 0.3 | 3.0 ± 0.5 |
| Infarktgewebe | 3 | 531 ± 180 (45 %) | 0.4 ± 0.2 (36 %) | 1.2 ± 0.4 (40 %) |
| rel.Verlust (%) | | 55 | 64 | 60 |

* Feuchtgewicht

Tab. 2: Creatin- und Creatinphosphatgehalt sowie Aktivität der Creatinkinase (CK) im Skelett- und Herzmuskel und der jeweilige relative Verlust nach einem Herzinfarkt.

## II. Analytischer Teil

Bestimmung von Creatin im Urin

### Beispiel 1

(Farbreagenz: 4-AAP/TOOS)

a) Reagenzien:

$a_1$) 100 mM Kaliumphosphatpuffer pH = 7.9
0.15 mM 4-AAP
10 mM TOOS
10 /uM Kaliumhexacyanoferrat II
3 kU/l Sarcosin-Oxidase
3 kU/l Peroxidase
10 kU/l Ascorbat-Oxidase

$a_2$) 100 mM Kaliumphosphatpuffer pH = 7.9
100 kU/l Creatinase

b) Testdurchführung:

Messung gegen Luft

T = 37° C    d = 1 cm    $\lambda$ = Hg 578 nm

in Zentrifugen-Röhrchen pipettieren:

| | Probenwert PW | Probenleerwert PLW |
|---|---|---|
| Reagenz $a_1$ | 1.00 ml | 1.00 ml |
| Reagenz $a_2$ | 100 /ul | - |
| $H_2O$ | - | 100 /ul |
| Probe* | 50 /ul | 50 /ul |
| mischen, 20 min. inkubieren, anschließend $E_{PW}$ und $E_{PLW}$ messen, $E_P = E_{PW} - E_{PLW}$. | | |

* Urin 1 + 9 mit 0.9 % NaCl verdünnen.

Berechnung: Zur Berechnung anstelle von Proben Creatin-Standard mit 1 mg/dl einsetzen.

$$C = \frac{E_p}{E_{St}} \times 10 \text{ (mg/dl)}$$

Beispiel 2

(Farbreagenz: S-MBTH/TOOS)

$a_1$) 100 mM Kaliumphosphatpuffer pH = 7.9
0.15 mM S-MBTH
2 mM TOOS
10 µM Kaliumhexacyanoferrat II
3 kU/l Sarcosin-Oxidase
3 kU/l Peroxidase
10 kU/l Ascorbat-Oxidase

$a_2$) 100 mM Kaliumphosphatpuffer pH = 7.9
100 kU/l Creatinase

b) Testdurchführung:

Messung gegen Luft

T = 37° C    d = 1 cm    $\lambda$ = Hg 578 nm

in Zentrifugen-Röhrchen pipettieren:

| | Probenwert PW | Probenleerwert PLW |
|---|---|---|
| Reagenz $a_1$ | 1.00 ml | 1.00 ml |
| Reagenz $a_2$ | 100 µl | - |
| $H_2O$ | - | 100 µl |
| Probe* | 20 µl | 20 µl |
| mischen, 20 min. inkubieren, anschließend $E_{PW}$ und $E_{PLW}$ messen, $E_P = E_{PW} - E_{PLW}$. | | |

* Urin 1 + 9 mit 0.9 % NaCl verdünnen.

Berechnung: Zur Berechnung anstelle von Proben Creatin-Standard mit 1 mg/dl einsetzen.

$$C = \frac{E_P}{E_{St}} \times 10 \text{ (mg/dl)}$$

Beispiel 3

(Farbreagenz: S-MBTH/TBHB)

a) Reagenzien:

$a_1$) 100 mM Kaliumphosphatpuffer pH = 7.9
0.15 mM S-MBTH
10 mM TBHB
10 µM Kaliumhexacyanoferrat II
3 kU/l Sarcosin-Oxidase
3 kU/l Peroxidase
10 kU/l Ascorbat-Oxidase

$a_2$ 100 mM Kaliumphosphatpuffer pH = 7.9
100 kU/l Creatinase

b) Messung gegen Luft

$T = 37^{\circ}$ C    d = 1 cm    $\lambda$ = Hg 546 nm

in Zentrifugen-Röhrchen pipettieren:

| | Probenwert PW | Probenleerwert PLW |
|---|---|---|
| Reagenz $a_1$ | 1.00 ml | 1.00 ml |
| Reagenz $a_2$ | 100 $\mu$l | - |
| $H_2O$ | - | 100 $\mu$l |
| Probe* | 25 $\mu$l | 25 $\mu$l |
| mischen, 20 min. inkubieren, anschließend $E_{PW}$ und $E_{PLW}$ messen, $E_P = E_{PW} - E_{PLW}$. | | |

* Urin 1 + 9 mit 0.9 % NaCl verdünnen.

Berechnung: Zur Berechnung anstelle von Proben Creatin-Standard mit 1 mg/dl einsetzen.

$$C = \frac{E_P}{E_{St}} \times 10 \text{ (mg/dl)}$$

Die Bestimmung verläuft bis zu einer Konzentration von 20 mg/100 ml Urin linear.

Analog kann die Bestimmung von Creatin im Blut, Serum, Plasma, ihren Ultrafiltraten oder Gewebsextrakten durchgeführt werden.

Die Figuren 1 und 2 zeigen die Resultate der Creatinbestimmung im Urin bzw. Serum im Vergleich zu der Aktivität der CK bzw. CK-MB im Serum eines typischen Herzinfarktpatienten bis zu 36 Stunden nach Auftreten der Symptome.

## Patentansprüche

1. Verfahren zum Nachweis von Schädigungen oder Erkrankungen des Herzens, dadurch gekennzeichnet, daß mindestens ein niedermolekularer Metabolit in der Probe einer Körperflüssigkeit bestimmt wird.

2. Verfahren nach Anspruch 1, dadurch gekennzeichnet, daß es sich bei den Schädigungen oder Erkrankungen des Herzens um die Formen akuter Herzinfarkt, Mikronekrosen, akute und stumme Myokardischämie oder Herzkontusion nach stumpfen Thoraxtraumen handelt.

3. Verfahren nach einem der Ansprüche 1 und 2, dadurch gekennzeichnet, daß es sich bei dem niedermolekularen Metaboliten um Creatin, um ein im Stoffwechsel vorhandenes Vor- oder Folgeprodukt oder einen natürlichen oder künstlichen Artefakt des Creatins handelt.

4. Verfahren nach Anspruch 3, dadurch gekennzeichnet, daß es sich bei dem niedermolekularen Metaboliten um Creatinphosphat oder Sarcosin handelt.

5. Verfahren nach einem der Ansprüche 1 bis 4, dadurch gekennzeichnet, daß es sich bei Körperflüssigkeiten um Urin, Blut, Serum, Plasma, Speichel oder einen Gewebeextrakt handelt.

6. Verfahren nach einem der Ansprüche 1 bis 5, dadurch gekennzeichnet, daß der niedermolekulare Metabolit im Zeitraum von 1 bis 24 Stunden nach Eintritt der Herzschädigung bestimmt wird.

7. Verfahren nach einem der Ansprüche 1 bis 6, dadurch gekennzeichnet, daß der niedermolekulare Metabolit im Zeitraum von 2 - 8 Stunden nach Eintritt der Herzschädigung bestimmt wird.

8. Verfahren nach Anspruch 3, dadurch gekennzeichnet, daß das Creatin enzymatisch mittels Sarcosin-Oxidase unter Bildung von Wasserstoffperoxid, welches mit einem Wasserstoff-Donor und einem Elektronen- oder Radikal-Akzeptor als farbbildendes System bzw. einem Leukofarbstoff bestimmt wird.

9. Verfahren nach Anspruch 8, dadurch gekennzeichnet, daß der Wasserstoff-Donor ein Anilin-Derivat ist.

10. Verfahren nach Anspruch 9, dadurch gekennzeichnet, daß das Anilinderivat eine Verbindung der folgenden allgemeinen Formel ist:

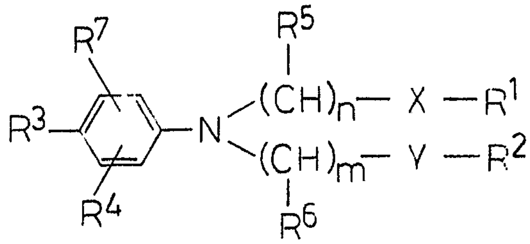

in der

| | |
|---|---|
| n und m | die Zahlen von 1 bis 3 bedeuten können, |
| X und Y | einen Valenzstrich, einen Arylen- oder Hydroxyalkylenrest mit 1 - 6 C-Atomen, |
| $R^1$ und $R^2$ | Wasserstoff, eine Hydroxy-, Alkyl- oder Alkoxygruppe mit 1 - 6 C-Atomen, eine Carboxyl-, Sulfonsäure-, eine Acetamido- oder eine P(O)(OR)(OR')-Gruppe sein kann, |

worin

| | |
|---|---|
| P | ein Phosphoratom bedeutet und |
| R | Wasserstoff oder eine Alkylgruppe mit 1 - 6 C-Atomen, |
| R' | eine Hydroxy-, eine Aryl-, Alkyl- oder Alkoxygruppe mit 1 - 6 C-Atomen sein kann, |
| $R^3$ | Wasserstoff, Halogen, eine Carboxyl- oder eine Sulfonsäuregruppe sein kann, |

wobei Halogen, Jod, Brom und bevorzugt Chlor und Fluor umfaßt,

| | |
|---|---|
| $R^4$ und $R^7$ | Wasserstoff, eine bevorzugt in meta-Stellung stehende Alkoxy- oder Alkylgruppe mit 1 - 6 C-Atomen, |
| $R^5$ und $R^6$ | für Wasserstoff, eine Aryl- oder Alkylgruppe mit 1 - 6 C-Atomen stehen und |
| $R^5$ und $R^7$ | bzw. $R^6$ und $R^4$ für den Fall, daß die beiden Substituenten orthoständig zueinander stehen, zusammen eine gesättigte oder ungesättigte Kohlenwasserstoffkette mit 2 - 6 C-Atomen bilden können, die durch Hydroxy- oder Oxogruppen substituiert sein kann, |

sowie ihre Salze mit Säuren und Basen.

11. Verfahren nach Anspruch 10, dadurch gekennzeichnet, daß das Anilin-Derivat N-Ethyl-N-(2-hydroxy-3-sulfopropyl)-m-toluidin (TOOS) oder (N-Methylanilino)-methanphosphonsäure ist.

12. Verfahren nach einem der Ansprüche 8 bis 11, dadurch gekennzeichnet, daß der Elektronen- oder Radikal-Akzeptor 4-Aminophenazon (4-AAP), 3-Methyl-2-benzothiazolinonhydrazon (MBTH) oder 3-Methyl-2-benzothiazolinonhydrazon-6-sulfonsäure (S-MBTH) ist.

13. Verfahren nach einem der Ansprüche 8 bis 12, dadurch gekennzeichnet, daß das Creatin-Nachweissystem die Enzyme Creatinamidinohydrolase und Sarcosinoxidase beinhaltet.

14. Verfahren nach Anspruch 13, dadurch gekennzeichnet, daß man einen zusätzlichen Bestimmungswert heranzieht, der durch Umsetzung erhalten wird, die entweder vorgeschaltet oder parallel durchgeführt wird, aber ohne Anwesenheit von Creatinamidinohydrolase.

15. Verfahren zur Bestimmung von Creatin in einer Körperflüssigkeit mit einem farbbildenden System, welches einen Wasserstoff-Donor und einen Elektronen- oder Radikal-Akzeptor enthält, dadurch gekennzeichnet, daß der Wasserstoff-Donor ein Anilin-Derivat ist.

16. Verfahren nach Anspruch 15, dadurch gekennzeichnet, daß die Körperflüssigkeit Urin, Blut, Serum, Plasma, Speichel oder ein Gewebeextrakt ist.

17. Verfahren nach Anspruch 16, dadurch gekennzeichnet, daß das Anilinderivat eine Verbindung der folgenden allgemeinen Formel ist:

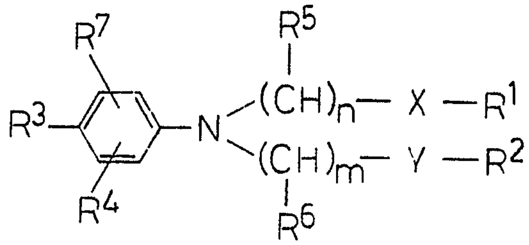

in der

| | |
|---|---|
| n und m | die Zahlen von 1 bis 3 bedeuten können, |
| X und Y | einen Valenzstrich, einen Arylen- oder Hydroxyalkylenrest mit 1 - 6 C-Atomen, |
| $R^1$ und $R^2$ | Wasserstoff, eine Hydroxy-, Alkyl- oder Alkoxygruppe mit 1 - 6 C-Atomen, eine Carboxyl-, Sulfonsäure-, eine Acetamido- oder eine P(O)(OR)(OR')-Gruppe sein kann, |
| worin | |
| P | ein Phosphoratom bedeutet und |
| R | Wasserstoff oder eine Alkylgruppe mit 1 - 6 C-Atomen, |
| R' | eine Hydroxy-, eine Aryl-, Alkyl- oder Alkoxygruppe mit 1 - 6 C-Atomen sein kann, |
| $R^3$ | Wasserstoff, Halogen, eine Carboxyl- oder eine Sulfonsäuregruppe sein kann, |

wobei Halogen, Jod, Brom und bevorzugt Chlor und Fluor umfaßt,

$R^4$ und $R^7$ Wasserstoff, eine bevorzugt in meta-Stellung stehende Alkoxy- oder Alkylgruppe mit 1 - 6 C-Atomen,

$R^5$ und $R^6$ für Wasserstoff, eine Aryl- oder Alkylgruppe mit 1 - 6 C-Atomen stehen und

$R^5$ und $R^7$ bzw. $R^6$ und $R^4$ für den Fall, daß die beiden Substituenten orthoständig zueinander stehen, zusammen eine gesättigte oder ungesättigte Kohlenwasserstoffkette mit 2 - 6 C-Atomen bilden können, die durch Hydroxy- oder Oxogruppen substituiert sein kann,

sowie ihre Salze mit Säuren und Basen.

18. Verfahren nach Anspruch 17, dadurch gekennzeichnet, daß das Anilin-Derivat N-Ethyl-N-(2-hydroxy-3-sulfopropyl)-m-toluidin (TOOS) oder (N-Methylanilino)-methanphosphonsäure ist.

19. Verfahren nach einem der Ansprüche 15 bis 18, dadurch gekennzeichnet, daß der Elektronen- oder Radikal-Akzeptor 4-Aminophenazon (4-AAP), 3-Methyl-2-benzothiazolinonhydrazon (MBTH) oder 3-Methyl-2-benzothiazolinonhydrazon-6-sulfonsäure (S-MBTH) ist.

20. Reagenz zur Bestimmung von Creatin in einer Körperflüssigkeit, enthaltend ein farbbildendes System mit einem Wasserstoff-Donor und einem Elektronen- oder Radikal-Akzeptor, dadurch gekennzeichnet, daß der Wasserstoff-Donor ein Anilin-Derivat ist.

21. Reagenz nach Anspruch 20, dadurch gekennzeichnet, daß die Körperflüssigkeit Urin, Blut, Serum, Plasma, Speichel oder ein Gewebeextrakt ist.

22. Reagenz nach einem der Ansprüche 20 und 21, dadurch gekennzeichnet, daß das Anilindervat eine Verbindung der allgemeinen Formel ist:

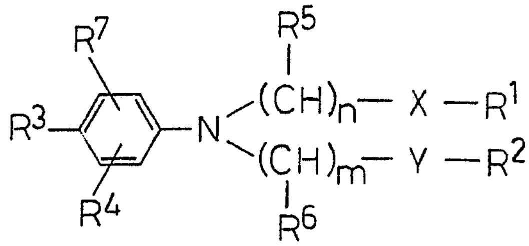

in der

n und m die Zahlen von 1 bis 3 bedeuten können,

X und Y einen Valenzstrich, einen Arylen- oder Hydroxyalkylenrest mit 1 - 6 C-Atomen,

$R^1$ und $R^2$ Wasserstoff, eine Hydroxy-, Alkyl- oder Alkoxygruppe mit 1 - 6 C-Atomen, eine Carboxyl-, Sulfonsäure-, eine Acetamido- oder eine P(O)(OR)(OR')-Gruppe sein kann,

worin

P ein Phosphoratom bedeutet und

R Wasserstoff oder eine Alkylgruppe mit 1 - 6 C-Atomen,

R' eine Hydroxy-, eine Aryl-, Alkyl- oder Alkoxygruppe mit 1 - 6 C-Atomen sein kann,

$R^3$ Wasserstoff, Halogen, eine Carboxyl- oder eine Sulfonsäuregruppe sein kann,

wobei Halogen, Jod, Brom und bevorzugt Chlor und Fluor umfaßt,

$R^4$ und $R^7$ Wasserstoff, eine bevorzugt in meta-Stellung stehende Alkoxy- oder Alkylgruppe mit 1 - 6 C-Atomen,

$R^5$ und $R^6$ für Wasserstoff, eine Aryl- oder Alkylgruppe mit 1 - 6 C-Atomen stehen und

$R^5$ und $R^7$ bzw. $R^6$ und $R^4$ für den Fall, daß die beiden Substituenten orthoständig zueinander stehen, zusammen eine gesättigte oder ungesättigte Kohlenwasserstoffkette mit 2 - 6 C-Atomen bilden können, die durch Hydroxy- oder Oxogruppen substituiert sein kann,

sowie ihre Salze mit Säuren und Basen.

23. Reagenz nach einem der Ansprüche 20 und 22, dadurch gekennzeichnet, daß das Anilin-Derivat N-Ethyl-N-(2-hydroxy-3-sulfopropyl)-m-toluidin (TOOS) oder (N-Methylanilino)-methanphosphonsäure ist.

24. Reagenz nach einem der Ansprüche 20 bis 23, dadurch gekennzeichnet, daß der Elektronen- oder Radikal-Akzeptor 4-Aminophenazon (4-AAP), 3-Methyl-2-benzothiazolinonhydrazon (MBTH) oder 3-Methyl-2-benzothiazolinonhydrazon-6-sulfonsäure (S-MBTH) ist.

25. Reagenz nach einem der Ansprüche 20 bis 24, dadurch gekennzeichnet, daß es Creatinamidinohydrolase, Sarcosinoxidase, Peroxidase und als Wasserstoff-Donor N-Ethyl-N-(2-hydroxy-3-sulfopropyl)-m-toluidin (TOOS) oder (N-Methylanilino)methanphosphonsäure und als Elektronen- oder Radial-Akzeptor 4-Aminophenazon (4-AAP), 3-Methyl-2-benzothiazolinonhydrazon (MBTH) oder 3-Methyl-2-benzothiazolinonhydrazon-6-sulfonsäure (S-MBTH) enthält.

26. Verwendung des Verfahrens nach einem der Ansprüche 1 bis 19 zur Verlaufskontrolle bei Herzoperationen und/oder bei Indikationen zur Herztherapie, z. B. bei Thrombolyse, oder zur weiteren klinischen Beobachtung nach einem Herzinfarkt.

1/2

0243901

# Fig.1

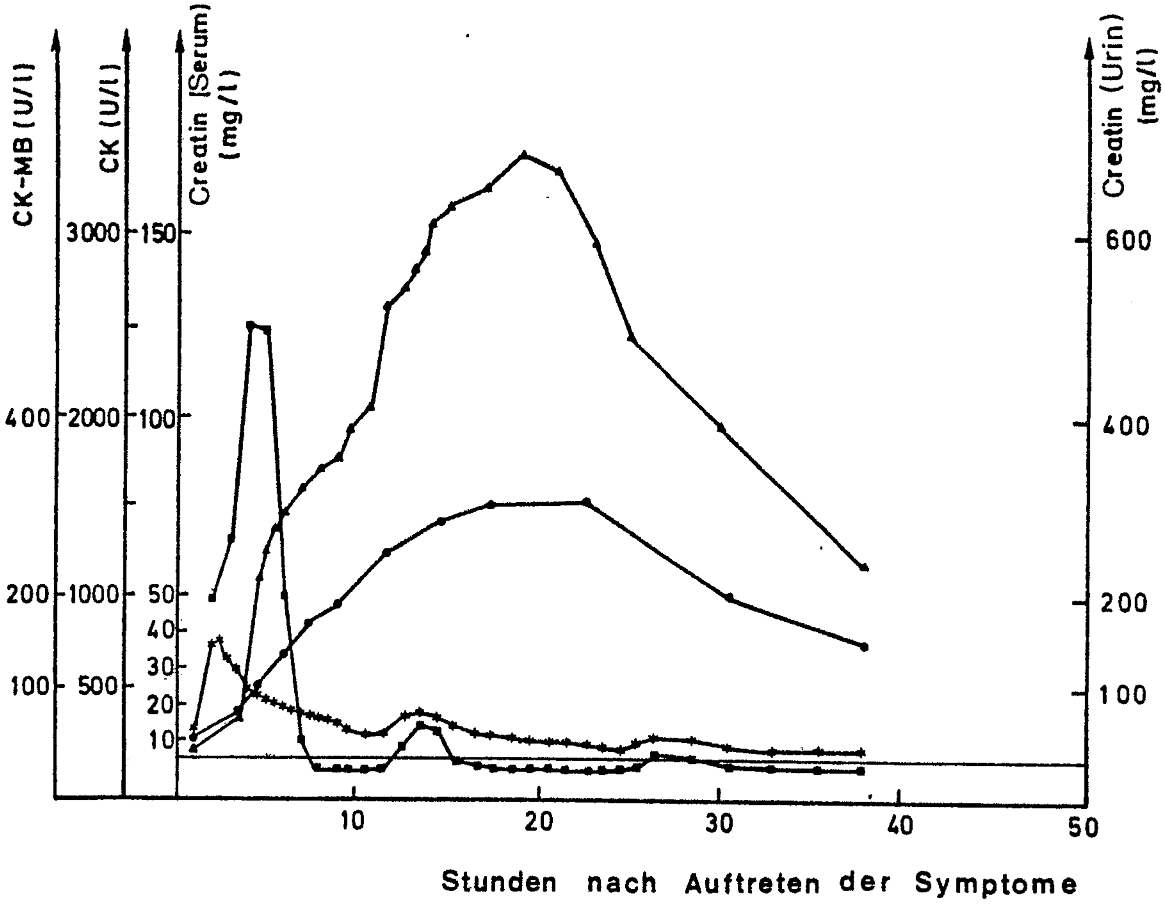


Verlauf der Creatinkonzentration und der Creatinkinase-aktivität eines Herzinfarktpatienten

■ = Creatinkonzentration im Urin (mg/L)

✶ = Creatinkonzentration im Serum (mg/L)

● = Aktivität der Creatinkinase-Untereinheit MB im Serum (U/L)

▲ = Aktivität der Creatinkinase (U/L)

Fig.2

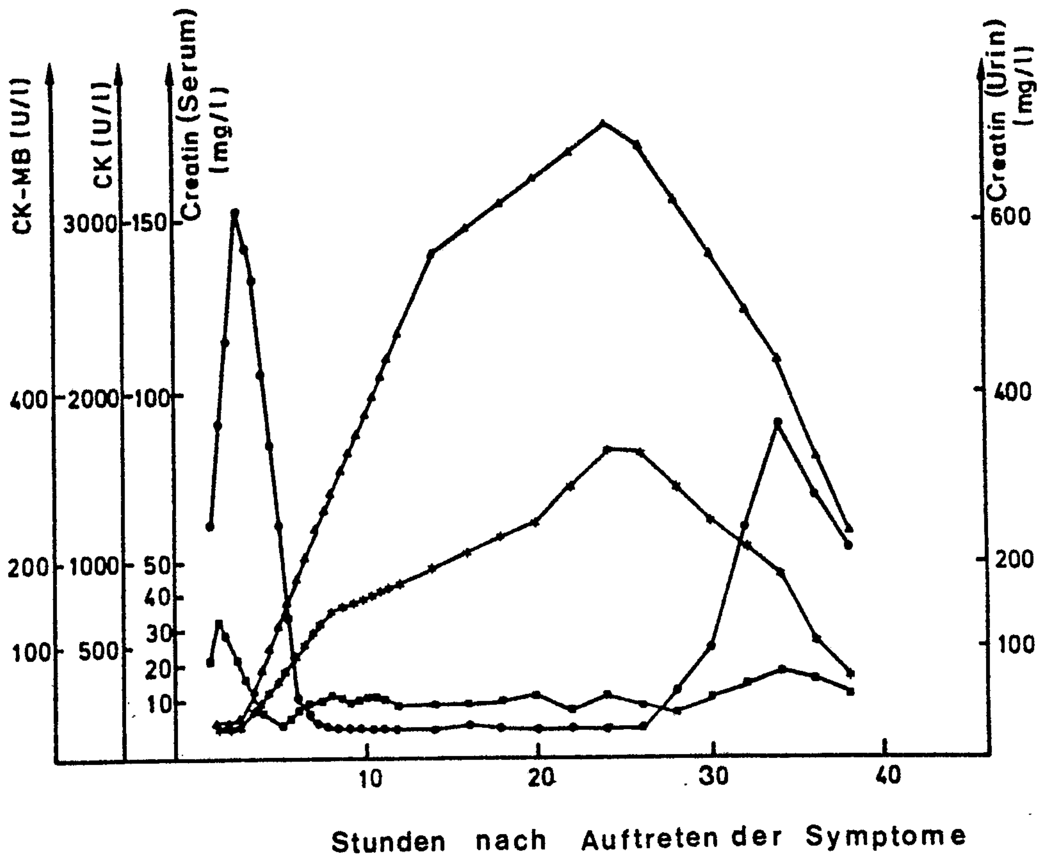


Verlauf der Creatinkonzentration und der Creatinkinase-aktivität eines Herzinfarktpatienten

■ = Creatinkonzentration im Serum (mg/L)

✶ = Aktivität der Creatinkinase-Untereinheit MB im Serum (U/L

● = Creatinkonzentration im Urin (mg/L)

▲ = Aktivität der Creatinkinase im Serum (U/L)

0243901

Europäisches Patentamt

# EUROPÄISCHER RECHERCHENBERICHT

Nummer der Anmeldung

EP 87 10 6028

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | CLINICAL CHEMISTRY, Band 32, Nr. 8, August 1986, Seite 1611, Winston Salem, US; J. DELANGHE et al.: "Early diagnosis of acute myocardial infarction by enzymatic urinary creatine determination" * Das ganze Dokument * | 1-3,5-7,12-14,19,24-26 | C 12 Q 1/26<br>C 12 Q 1/28<br>C 12 Q 1/34 |
| X | CHEMICAL ABSTRACTS, Band 101, Nr. 13, 24. September 1984, Seite 301, Zusammenfassung Nr. 106705c, Columbus, Ohio, US; M. SUZUKI et al.: "A new enzymic determination of serum creatine", & CLIN. CHIM. ACTA 1984, 140(3), 289-94 | 3,8,13 | |
| Y | Idem | 8-13, 15-25 | **RECHERCHIERTE SACHGEBIETE (Int. Cl.4)** |
| | | | C 12 Q |
| D,Y | EP-A-0 068 356 (BOEHRINGER MANNHEIM) * Seite 3, Zeilen 26-35; Seiten 4,6; Ansprüche * | 8-13, 15-25 | |
| D,Y | EP-A-0 168 035 (BOEHRINGER MANNHEIM) * Seiten 6,7; Ansprüche * | 10,17, 22 | |

--- -/-

Der vorliegende Recherchenbericht wurde für alle Patentansprüche erstellt.

| Recherchenort | Abschlußdatum der Recherche | Prüfer |
|---|---|---|
| DEN HAAG | 14-07-1987 | MEYLAERTS H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0243901

Europäisches Patentamt

# EUROPÄISCHER RECHERCHENBERICHT

Nummer der Anmeldung

EP 87 10 6028

| EINSCHLÄGIGE DOKUMENTE | | | Seite 2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | EP-A-0 121 254 (WAKO PURE CHEMICAL INDUSTRIES)<br>* Seite 13, Zeilen 15-18; Seite 14; Seite 6, Zeilen 2-9; Ansprüche 1,5; Seite 9, Zeilen 15-25; Seite 10, Tabel 2 *<br>--- | 3,8-10,12,13,15-17 | |
| A | US-A-4 105 499 (J.Y. KIYASU)<br>--- | | |
| A | CHEMICAL ABSTRACTS, Band 103, Nr. 9, 2. September 1985, Seite 467, Zusammenfassung Nr. 69187d, Columbus, Ohio, US; J. LARSSON et al.: "Intracellular energy substrates in trauma", & ACTA CHIR. SCAND. SUPPL. 1985, 522, 171-81<br>----- | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 14-07-1987 | MEYLAERTS H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument